# FASCICULE DE BREVET EUROPEEN

(11) **EP 2 958 547 B1**
(45) Date de publication et mention de la délivrance du brevet: **23.10.2019**
(21) Numéro de dépôt: 14705345.8
(22) Date de dépôt: 18.02.2014
(51) Int. Cl.: A61K 8/49, A61Q 19/08

(54) **UTILISATION COSMÉTIQUE DE LA QUEUINE**
KOSMETISCHE VERWENDUNG VON QUEUIN
COSMETIC USE OF QUEUINE

(30) Priorité: 21.02.2013 FR 1351486
(43) Date de publication de la demande: 30.12.2015
(73) Titulaire: Amabiotics, 75003 Paris (FR)
(72) Inventeur: DANCHIN, Antoine, F-75013 Paris (FR); SEKOWSKA, Agnieszka, F-75013 Paris (FR)
(74) Mandataire: Vial, Lionel
(86) Numéro de dépôt international: PCT/EP2014/053143
(87) Numéro de publication internationale: WO 2014/128126

(56) Documents cités:
- US-A- 5 312 900
- US-A1- 2004 091 509
- CHANDRAMANI PATHAK ET AL: "Queuine promotes antioxidant defence system by activating cellular antioxidant enzyme activities in cancer", BIOSCIENCE REPORTS, vol. 28, no. 2, 1 avril 2008 (2008-04-01), page 73, XP055092211, ISSN: 0144-8463, DOI: 10.1042/BSR20070011
- Chandramani Pathak: "Modulation in the activity of lactate dehydrogenase and level of c-Myc and c-Fos by modified base queuine in cancer", Cancer Biology & Therapy, 1 janvier 2008 (2008-01-01), pages 85-91, XP055092222, United States DOI: 10.4161/cbt.7.1.5133 Extrait de l'Internet: URL:http://www.landesbioscience.com/journa ls/cbt/article/5133/ [extrait le 2013-12-06]
- Domenico Fusco ET AL: "Effects of antioxidant supplementation on the aging process", Clinical interventions in aging, 1 janvier 2007 (2007-01-01), pages 377-387, XP055092277, New Zealand Extrait de l'Internet: URL:http://www.pubmedcentral.nih.gov/artic lerender.fcgi?artid=2685276&tool=pmcentrez &rendertype=abstract [extrait le 2013-12-06]

## Description

### Domaine de l'Invention

La présente invention est relative au domaine de la cosmétique, en particulier aux produits pour prévenir ou lutter contre le vieillissement de la peau et des phanères.

### Contexte de l'Invention

Comme tissu protecteur et frontière d'échange, la peau se renouvelle rapidement tout au long de la vie. Le temps qui s'écoule résulte inévitablement en un vieillissement de la peau. Un vieillissement prématuré peut même subvenir comme conséquence d'agressions environnementales multiples. En tant qu'interface avec l'environnement externe, la peau est constamment soumise à des dommages résultant de toutes sortes d'agressions physico-chimiques : changement de température, humidité, lumière, pollution, etc. Les cellules de la peau vieillissent, puis deviennent sénescentes et meurent après environ 80 divisions. Pendant ce processus, la peau perd son épaisseur et son élasticité, faisant apparaître des rides, mais également son rôle protecteur, en particulier contre les radiations UV du jour. Elle peut également avoir une pigmentation irrégulière (taches de vieillesse).

Les causes moléculaires du vieillissement de la peau impliquent tous les compartiments de la peau. Comme dans les autres tissus, des protéines induites par le stress jouent un rôle protecteur central, en particulier les protéines chaperonnes qui permettent le repliement convenable des protéines et corrigent les défauts de ce repliement et les protéases qui dégradent les protéines chimiquement altérées ou mal-repliées. Le processus de traduction et de repliement est progressivement moins efficace lorsque les cellules vieillissent, résultant ainsi en une production et une sensibilité accrue aux modifications oxydantes accidentelles et aux glycations. Des espèces réactives d'oxygène altèrent les acides aminés (en particulier, cystéine, histidine, méthionine, tyrosine et tryptophane) et les squelettes protéiques. Elles altèrent aussi en amont les processus qui conduisent à la synthèse de nouvelles protéines.

L'accumulation des agrégats protéiques pendant le processus de vieillissement submerge progressivement la machinerie de contrôle de la qualité des protéines cellulaires. Ce vieillissement de la peau implique une dégradation de la matrice extracellulaire à la fois un niveau des couches de l'épiderme et du derme, laissant des signes visibles du vieillissement à la surface de la peau et modifiant les propriétés physiques de celle-ci.

La conséquence globale est également une altération de l'activité enzymatique normale et une agrégation de protéines entières, entraînant des signes visibles comme une sécheresse irrégulière, une pigmentation irrégulière, des rides profondes, un teint cireux et/ou parcheminé, un relâchement de la peau, etc.

Trois processus permettent aux cellules de surmonter le vieillissement : la néo-synthèse, la réparation et la dégradation suivie de re-synthèse. A long terme, les deux derniers processus sont essentiels car ils permettent un rajeunissement des cellules.

Ainsi, il existe un besoin et une forte demande de nouveaux soins cosmétiques pour lutter contre le vieillissement de la peau.

### Description de l'Invention

Les inventeurs ont découvert de manière tout à fait surprenante qu'un apport exogène de queuine remédie aux effets délétères des stress subis de façon inévitable par la peau, retardant ainsi l'apparition des signes du vieillissement. La queuine contribue ainsi de façon importante à l'entretien des cellules. La queuine a un effet protecteur lors de stress multiples et répétés. Plus particulièrement, la présente invention a exploité la nécessité d'optimiser le processus de re-synthèse des protéines en fournissant aux cellules de la queuine. En effet, cette molécule est nécessaire au déroulement optimal de la synthèse protéique et l'organisme ne sait pas la fabriquer. Il a été découvert dans le contexte de la présente invention qu'un apport exogène de queuine contribue de manière surprenante à la résistance des cellules de la peau. Ainsi, la présente invention est relative à une composition cosmétique comprenant de la queuine, un précurseur ou dérivé de celle-ci selon la revendication 1, en tant que principe actif. La présente invention a plus particulièrement pour objet une composition cosmétique pour application topique.

La présente invention est également relative à l'utilisation de la queuine, un précurseur ou dérivé de celle-ci selon la revendication 1, en tant que principe actif dans une composition cosmétique, et tout particulièrement une composition cosmétique pour application topique.

L'invention a également pour objet une utilisation cosmétique de queuine, ou d'un précurseur ou dérivé de celle-ci en tant que principe actif pour diminuer ou prévenir les signes de vieillissement de la peau et/ou des phanères. Par phanères on entend de préférence les cheveux et les ongles.

L'utilisation ou composition selon l'invention est tout particulièrement destinée à diminuer ou prévenir les rides et ridules et/ou le flétrissement de la peau et/ou le manque d'élasticité et/ou de tonus de la peau et/ou l'amincissement de la peau et/ou le teint cireux et/ou parcheminé et/ou les irrégularités dans la texture de la peau et les irrégularités dans la pigmentation comme les taches de vieillesse.

L'invention concerne en outre une méthode de préparation d'une composition cosmétique comprenant l'ajout de la queuine, un précurseur ou dérivé de celle-ci aux ou à des composants de la composition cosmétique et la récupération de la composition cosmétique obtenue.

La présente invention concerne également un procédé de traitement cosmétique pour prévenir et/ou traiter les signes de vieillissement de la peau et/ou des phanères, comprenant l'application sur la peau et/ou les phanères d'une composition cosmétique selon l'invention.

Dans un mode de réalisation particulier, la queuine, le précurseur ou dérivé de celle-ci est choisi dans le groupe consistant en la queuine, la queuosine, l'époxyqueuosine et l'époxyqueuine. Parmi ces dérivés se trouvent les dérivés glycosylés de la queuine et de la queuosine, comme la mannosylqueuine, la galactosylqueuine, ainsi que des dérivés aminoacylés comme la glutamylqueuine. La queuine, un précurseur ou dérivé de celle-ci peut être sous forme purifiée ou sous forme d'extrait bactérien ou d'extrait ou sève de plante, ledit extrait ou ladite sève étant riche ou enrichi(e) en queuine, précurseur ou dérivé de celle-ci. Dans un mode de réalisation tout particulièrement préféré, la composition cosmétique comprend de la queuine.

De préférence, la queuine, le précurseur ou dérivé de celle-ci est présent dans la composition cosmétique dans une quantité de 0,1 µg à 100 µg par ml ou g de composition cosmétique, de préférence de 0,5 à 10 µg par ml ou g de composition cosmétique et de manière encore plus préférée de 1 à 5 µg par ml ou g de composition cosmétique. Pour les dérivés à libération lente, les concentrations sont calculées de façon à ce que la vitesse de libération remplisse à chaque instant les conditions précitées en conformité avec le processus physico-chimique de libération. Facultativement, la composition cosmétique peut comprendre la queuine, le précurseur ou dérivé de celle-ci dans une quantité d'au moins 0,1 µg à 100 µg par ml ou g de composition cosmétique, de préférence d'au moins 0,5 à 10 µg par ml ou g de composition cosmétique et de manière encore plus préférée d'au moins 1 à 5 µg par ml ou g de composition cosmétique

La composition cosmétique peut se présenter sous forme de sérum, de lotion, de crème, de lait, de gel aqueux ou huileux, d'hydrogel, de microémulsion, de nanoémulsion, de masque, de bâton, de patch, d'huile, d'onguent, de cire, d'une mousse, de tonique, d'eau de soin, de baume, de fond de teint, de spray, d'ombre à paupière, de crème amincissante, de rouge à lèvre, d'une pâte, d'une pommade ou d'un shampoing ou après-shampoing, ou de toute forme convenable homogène ou hétérogène permettant l'usage de la queuine et de ses dérivés.

### Queuine, précurseurs et dérivés de celle-ci.

La queuine est également connue sous le nom chimique suivant : 2-amino-5-((((1s,4s,5r)-4,5-dihydroxy-2-cyclopentèn-1-yl)amino)méthyl)-1,7-dihydro-4h-pyrrolo(2,3-d)pyrimidin-4-one.

### (Numéro CAS : 72496-59-4)

L'époxyqueuine est également connue sous le nom chimique suivant : 7-(5-[(3,4-epoxy-2,5-dihydroxycyclopent-1-yl)amino]methyl)-7-deazaguanine 2-amino-5-({[(1R,2R,3R,4R,5S)-3,4-dihydroxy-6-oxabicyclo[3.1.0]hex-2-yl]amino}methyl)-3,7-dihydro-4H-pyrrolo[2,3-d]pyrimidin-4-one. (Numéro CID : 56927905) La queuosine est également connue sous le nom chimique suivant : 2-amino-5-[[[(1*S*,4*S*,5*R*)-4,5-dihydroxy-1-cyclopent-2-enyl]amino]méthyl]-7-[(2*R*,3*R*,4*S*,5*R*)-3,4-dihydroxy-5-(hydroxyméthyl)-2-tétrahydrofuranyl]-1*H*-pyrrolo[3,2-e]pyrimidin-4-one. (Numéro CAS : 57072-36-3)

L'époxyqueuosine est également connue sous le nom chimique suivant : 7-(5-[(3,4-epoxy-2,5-dihydroxycyclopent-1-yl)amino]methyl)-7-deazaguanosine2-amino-5-({[(1R,2R,3R,4R,5S)-3,4-dihydroxy-6-oxabicyclo[3.1.0]hex-2-yl]amino}methyl)-7-(beta-D-ribofuranosyl)-3,7-dihydro-4H-pyrrolo[2,3-d]pyrimidin-4-one (Numéro CID : 56927875)

Dans le contexte de la présente demande, un dérivé de la queuine fait référence à toute molécule ou macromolécules comprenant de la queuine dans une forme adaptée pour être utilisée par la peau ou les phanères. En particulier, la queuine peut être sous forme libre ou comme partie d'un complexe covalent ou ionique. Par exemple, elle peut être complexée avec un ribose pour former la queuosine, une galactosyl queuosine, une mannosyl queuosine ou une glutamyl queuosine. On peut également la considérer sous forme d'ARNt-queuosine ou d'un oligonucléotide comprenant la queuosine. Parmi ces dérivés se trouvent les dérivés glycosylés de la queuine et de la queuosine, comme la mannosylqueuine, la galactosylqueuine, ainsi que des dérivés aminoacylés comme la glutamylqueuine.

Dans le contexte de la présente demande, un précurseur de la queuine fait référence par exemple à son précurseur intermédiaire, l'époxyqueuine, que ce soit sous forme libre ou sous forme de complexe covalent avec des molécules ou macromolécules.

Un dérivé de queuine peut également être une queuine chimiquement modifiée qui peut être facilement métabolisée en queuine par des enzymes telles que celles présentes à la surface de la peau. D'autres dérivés de la queuine sont des dérivés qui libèrent la queuine lorsqu'ils sont appliqués à la surface de la peau ou des phanères et soumis à un traitement physicochimiques (par exemple, lumière UV naturelle ou artificielle).

De manière préférée, la queuine, ses précurseurs et dérivés peuvent être préparés par synthèse chimique. La synthèse chimique de la queuine est connue. Notamment, deux voies principales de synthèse ont été décrites (Barnett and Grubb, 2000, Tetrahedron 56, 9221-9225 ; Brooks et al, 2010, Tetrahedron Letters 51, 4163-4165) et peuvent être mises en œuvre par l'homme du métier.

De façon alternative, la queuine, ainsi que ses précurseurs et dérivés, peuvent être obtenus à l'aide de bactéries qui synthétisent de telles molécules.

Ainsi, la queuine peut être obtenue par purification à partir d'extraits de microorganismes, notamment bactériens.

De manière alternative, la queuine, ses précurseurs ou dérivés sont sous forme d'extraits bactériens, le cas échéant enrichis en queuine, ses précurseurs ou dérivés.

De préférence, l'extrait bactérien est un extrait de bactéries choisies parmi des bactéries Firmicutes comestibles, de préférence des souches de *Bacillus subtilis* ou de son voisin *Bacillus amyloliquifaciens,* mais également des *Bacillus cereus* non-pathogènes, des *Streptococcus thermophilus* produisant de la queuine, des *Staphylococcus epidermidis* non-pathogènes, et en général les Firmicutes non-pathogènes de préférence utilisés dans l'alimentation. Parmi les bactéries Gram-négatif, les probiotiques gamma-Protéobactéries, telles que *E. coli* Nissle 1917, ou des protéobactéries utilisées pour l'alimentation comme *Zymononas mobilis* sont préférées. Les cyanobactéries non-toxicogéniques sont également des sources de queuine préférées, en particulier les espèces comestibles *Arthrospira* (Spiruline).

Dans un autre mode de réalisation, la queuine, ainsi que ses précurseurs et dérivés, peuvent être obtenus à partir de plantes ayant extrait ces molécules dans leur environnement, après vérification de leur contenu en queuine ou en ses dérivés. Notamment, les plantes présentant des nodules avec des alpha-protéobactéries, notamment les espèces *Rhizobium, Mesorhizobium,* et *Sinorhizobium,* sont une excellente source de queuine. En outre, la sève de plante est également une source intéressante de queuine lorsque leur croissance a été assurée dans un environnement microbien riche en bactéries adaptées à la rhizosphère ou qu'elles ont été colonisées par des microorganismes épiphytes et endophytes synthétisant la queuine, en particulier des familles Bacteroidetes, Firmicutes, and Protéobacteries (par exemple, mais sans limitation, *Pseudomonas fluorescens* ou *Serratia marcescens*). Les extraits (feuilles, racines, fruits, écorce...) de plantes utilisées peuvent être des organismes couramment utilisés dans l'alimentation, en cosmétique, ou en médecine traditionnelle, par exemple des plantes aussi diverses que: *Acalypha indica, Acanthus ebracteatus, Aloe vera, Avena sativa, Cocos nucifera, Coffea arabica, Colocasia esculenta, Curcuma longa, Hippophae rhamnoides, Jasminum sambac, Juglans mandshurica, Matricaria recutita, Mesembryanthemum crystallinum, Opuntia ficus indica, Oryza sativa, Pittocaulon praecox, Plagiochila beddomei, Populus balsamifera, Psidium guajava, Scutellaria baicalensis, Vaccinium* spp., *Vitis vinifera,* ce en fonction de leur teneur en queuine qui devra être évaluée. Les plantes utilisées traditionnellement pour leur effet positif sur l'élevage, souvent de la famille des Fabacées, Solanacées ou Asteracées, mais comprenant un très grand nombre de plantes reconnues pour leur effet sur la qualité et la résistance de la peau, peuvent être retenues. Les cultures hors sol et sans apport microbien ou sans apport en queuine ne sont utilisées qu'à condition d'ajouter un apport exogène de la molécule. Toutefois les plantes rares ou protégées, comme *Leontopodium alpinum,* cultivées en laboratoire peuvent être choisies, si elles sont cultivées en présence de microorganismes producteurs de queuine, et après évaluation de leur aptitude à capter et concentrer la molécule.

Le contenu en queuine, ses précurseurs et dérivés, des extraits bactériens ou des extraits de plante est de préférence garanti. Pour cette raison, en raison de la dégradation de la flore microbienne associée aux extraits de plantes obtenus en culture intensive, ceux-ci ne sont préférentiellement utilisés dans les préparations qu'avec un apport exogène en queuine. De manière générale, les extraits bactériens ou de plantes peuvent être enrichis en queuine, ses précurseurs et dérivés.

### Composition cosmétique

Selon l'invention, de la queuine, un précurseur et/ou dérivé de queuine peut être utilisée en tant que principe actif dans une composition cosmétique destinée à prévenir ou traiter les signes du vieillissement cutané, chronologiques et/ou photo-induits, en particulier les signes de vieillissement prématuré touchant les personnes de 25-30 ans et se traduisant le plus souvent par l'apparition de ridules et/ou un aspect terne et/ou hétérogène du teint.

La composition selon l'invention comprend un milieu physiologiquement acceptable, c'est-à-dire compatible avec la peau et/ou les phanères. Il s'agit de préférence d'un milieu cosmétiquement acceptable, c'est-à-dire qui présente une couleur, une odeur et une consistance agréables et qui ne génère pas d'inconforts tels que des picotements, rougeurs ou autres, susceptibles de détourner le consommateur de son emploi.

Selon l'invention, la composition cosmétique peut être par exemple un produit de soin ou de maquillage, sous forme de sérum, de lotion, de crème, de lait, de gel aqueux ou huileux, d'hydrogel, de masque, de bâton, de patch, d'huile, d'onguent, de cire, d'une mousse, de tonique, d'eau de soin, de baume, de fond de teint, de spray, d'ombre à paupière, de rouge à lèvre, d'une pâte, d'une pommade ou d'un shampoing ou après-shampoing.

La composition cosmétique peut aussi être utilisée comme composition amincissante comprenant, outre de la queuine, un précurseur et/ou dérivé de queuine, un agent amincissant.

La composition étant destinée à une administration topique sur la peau et/ou les phanères, elle peut se présenter sous toute forme galénique classiquement utilisée pour une application topique. Notamment, elle peut se présenter avantageusement sous la forme de solutions aqueuses, hydroalcooliques, d'émulsions huile-dans-eau (H/E) ou eau-dans huile (E/H) ou multiple (triple : E/H/E ou H/E/H), ou des micro- ou nano-émulsions.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

De telles compositions peuvent également contenir les véhicules ou diluants pharmaceutiquement ou cosmétiquement acceptables, usuels, et appropriés notamment comme agent diluant, agent dispersant, agent gélifiant, émollient solide, des gommes, des résines, des solvants, des charges tels que des amidons modifiés et polymérisés, le dioxyde de titane, ou un stéarate métallique, des conservateurs, des huiles essentielles, des agents nacrés, des colorants, des absorbeurs d'odeur, des agents régulateurs du pH ou des agents neutralisants, des agents épaississants, des agents promoteurs d'absorption et en particulier l'éthanol et/ ou des phospholipides, des agents aromatisants ou parfumant, des pigments minéraux comme des oxydes de fer, des agents huileux comme des huiles ou des graisses d'origine végétale, des graisses d'origine animale, des huiles de synthèse, des huiles de silicone (cyclométhicone) des huiles fluorées, des esters d'alcool gras (alcool cetylique), des cires, des argiles modifiées, des bentones, des sels métalliques d'acide gras, de la silice hydrophobisée, des polyethylènes, du mica et/ou d'autres substances utilisées en cosmétique.

La composition cosmétique selon l'invention peut comprendre en outre d'autres molécules actives, telles que des vitamines, des filtres et écrans solaires, des actifs anti-âges, anti-rides, comme notamment des peptides, anti-oxydants, un actif éclaircissant, un actif auto-bronzant, un accélérateur de bronzage, un actif liftant, un amincissant, un actif raffermissant, un actif hydratant, un actif gommant, un séborégulateur, matifiant, etc. De préférence, la composition cosmétique pourra comprendre des actifs anti-âge, anti-rides, et/ou anti-oxydants, un actif liftant, un actif raffermissant, et/ou un actif hydratant. D'une manière générale, l'homme du métier sait choisir et adapter les quantités des molécules actives additionnelles de manière que les propriétés de la composition selon l'invention ne sont pas altérées par leur adjonction.

Dans un mode de réalisation préféré, la queuine, le précurseur ou dérivé de celle-ci est présent dans la composition cosmétique avec des co-facteurs ou vitamines, comme par exemple la vitamine C ou le tocophérol.

L'invention sera mieux comprise à la lecture des expérimentations et exemples qui suivent, et à l'examen des figures qui accompagnent. Ceux-ci sont donnés à titre illustratif et nullement limitatif de l'invention.
Les figures 1, 2 et 3 montrent la migration cellulaire dans une culture de fibroblastes incubée dans DMEM, 2,5% SVF à 0 heure (figure 1) et 24 heures (figure 2), ou dans DMEM, 10% SVF à 24 heures (figure 3) ;
Les figures 4 et 5 montrent la migration cellulaire dans une culture de fibroblastes incubée avec 10µg/ml de queuine dans 2,5% SVF à 24 heures (figure 4) ou 30µg/ml de queuine dans 2,5% SVF à 24 heures (figure 5) ;
Les figures 6 et 7 montrent la migration cellulaire dans une culture de fibroblastes incubée avec 10µg/ml de méthanol dans 2,5% SVF à 24 heures (figure 6) ou 30µg/ml de méthanol dans 2,5% SVF à 24 heures (figure 7).

### Expérimentations

Dans ces expérimentations, les effets de la queuine (cytotoxicité, prolifération, migration cellulaire) sont étudiés sur des fibroblastes de dermes humains adultes normaux.

### Matériel & méthodes

Le sérum de veau foetal SVF, Dutscher, P30-8100M (lot P130903) a été sélectionné pour une utilisation avec la queuine, qui ne contamine par le milieu.

Du méthanol est utilisé ici comme véhicule de la molécule active.

La solution mère de queuine a été réalisée à 15 mg/ml en méthanol et les dilutions sont ensuite réalisées en milieu DMEM (Dulbecco's Modified Essential Media) en présence d'une concentration variable de sérum de veau foetal (SVF) (DMEM+ 2,5% SVF + antibiotiques (Pénicilline/Streptomycine) + glutamine) : milieu appauvri avec 2,5% de SVF pour les expériences de viabilité, de prolifération et de migration cellulaire.

### Gammes de dilution

La gamme de dilution réalisée pour l'étude de toxicité/viabilité est la suivante:

**Tableau 1 : gamme de dilution pour l'étude de toxicité/viabilité**

| Concentrations (µg/ml) queuine | Pourcentage méthanol final (%) |
|---|---|
| 100 | 0,6 |
| 10 | 0,06 |
| 1 | 0,006 |
| 0,1 | 0,0006 |

Cette première étude a permis d'affiner la gamme de concentration étudiée.

La gamme de dilution réalisée pour l'étude de la cinétique de prolifération cellulaire est la suivante :

**Tableau 2 : gamme de dilution pour l'étude de la cinétique de prolifération cellulaire**

| Concentrations (µg/ml) queuine | Pourcentage méthanol final (%) |
|---|---|
| 30 | 0,18 |
| 10 | 0,06 |
| 3 | 0,018 |
| 1 | 0,006 |
| 0,3 | 0,0018 |

La gamme de dilution réalisée pour l'étude de migration est identique à celle de l'étude de cinétique, à l'exclusion du point 0,3 µg/ml.

### Produits utilisés:

**Tableau 3 : liste des produits utilisés**

| Produit | Fournisseur | Référence |
|---|---|---|
| DMEM w/o L-glutamine | Dutscher | L0101-500 |
| PBS 1X, w/o Ca Mg | PAA | H15-002 |
| Pénicilline/streptomycine | PAA | P11-010 |
| L-Glutamine | PAA | M11-004 |
| Trypsine-EDTA 10X | PAA | L11-003 |
| SVF | Dutscher | P30-8100M |
| BrdU | Roche | 11647229001 |
| MTT | Calbiochem | 475989 |
| Collagène | Nutacon | NBC_100A |
| HDFa | TebuBio | 106-05a |

### Protocoles expérimentaux

### 1. Lignée cellulaire

Type cellulaire : HDFa, fibroblastes dermiques humains adultes, femme de 39 ans, utilisés entre le passage 4 et le passage 6.

Pour toutes les expériences menées, les 3 témoins SVF suivants sont systématiquement ajoutés :
- DMEM sans sérum ;
- milieu appauvri en sérum (DMEM supplémenté par 2,5% SVF) ; et
- milieu complet (DMEM supplémenté par 10% SVF).

### 2. Évaluation de la cytotoxicité et de la prolifération cellulaire

Les HDFa utilisés sont ensemencés à raison de 5x10³ cellules/puits dans des plaques 96 puits (passage 4) dans 200 µl de milieu de culture sans SVF (DMEM supplémenté + 0% SVF). 24h après l'ensemencement, le milieu de culture est changé et les différentes dilutions du produit ou de son véhicule (méthanol) sont ajoutées sous 200 µl par puits, avec 3 puits par condition de culture.

3 témoins sont aussi étudiés (3 puits/témoin) :
- DMEM sans sérum ;
- milieu appauvri en sérum (DMEM supplémenté + 2,5% SVF) ; et
- milieu complet (DMEM supplémenté + 10% SVF).

### 3. Migration cellulaire

Les HDFa utilisés sont ensemencés en présence d'inserts de culture préalablement stérilisés, qui vont permettre de créer des zones acellulaires calibrées. 100 000 HDFa sous 600 µl et 30000 cellules sous 150 µl sont respectivement ajoutés à l'extérieur et à l'intérieur des inserts, en DMEM + 2,5% SVF. Après 5h d'incubation à 37°C, les inserts sont retirés et les puits lavés deux fois en PBS 1X afin de retirer toute cellule n'ayant pas adhéré. La molécule queuine est ensuite ajoutée à 30, 10, 3 ou 1 µg/ml en DMEM 2,5% SVF, et une gamme équivalente de méthanol est également réalisée, deux puits par condition. Les puits sont pris en photo à t=0h puis, les plaques sont ensuite incubées 24h. Les cellules sont alors fixées au paraformaldéhyde avant de réaliser les photos à 24h.

### Mesures et analyses des résultats

### 1. Quantification de la cytotoxité : test MTT

La prolifération des cellules est évaluée par un test colorimétrique (test MTT) après 1, 2 et 3 jours de culture. Ce test est basé sur la réduction du sel jaune de tétrazolium (MTT) [3-(4, 5-dimethylthiazol bromide] en un produit formazan bleu-violet par les réductases NADPH mitochondriales des cellules, à condition qu'elles soient vivantes. Les cristaux sont ensuite dissous par un mélange éthanol 100% - DMSO (v/v). La coloration du surnageant, proportionnelle à la quantité de cellules vivantes, est mesurée par lecture de la densité optique (DO) au spectrophotomètre à une longueur d'onde de 570 nm. Le test est effectué à J1, J2 et à J3 (J0 correspondant à l'addition des solutions à tester).

Les moyennes des DO des triplicats sont représentées dans un graphique pour chaque temps de la cinétique. Les écart-types sont représentés.

### 2. Quantification de la prolifération cellulaire : test BrdU

La prolifération des cellules est évaluée par un test de type ELISA (test BrdU, Bromodéoxyuridine) après 1, 2 et 3 jours de culture.

Le BrdU est un nucléoside synthétique analogue de la thymidine, qui peut être reconnu par un anticorps anti-BrdU. Il va être incorporé au cours de la réplication de l'ADN des cellules proliférantes. L'anticorps anti-BrdU est couplé à une enzyme qui en présence de son substrat, va le dégrader. Ainsi, la détection du BrdU est réalisée par test enzymatique colorimétrique.

La dégradation du substrat de l'enzyme va colorer le surnageant, proportionnellement à la quantité de BrdU incorporée. La mesure est alors faite par lecture de la densité optique (DO) au spectrophotomètre à une longueur d'onde de 450 nm. Le test est effectué à J1, J2 et à J3 (J0 correspondant au moment de l'addition des solutions à tester).

Les valeurs moyennes des DO, les écarts types et les figures de doses réponses sont détaillés ci-dessous pour chaque temps de la cinétique.

### 3. Quantification de la migration cellulaire : comparaison des images de migration par rapport au contrôle

Les images sont analysées avec le logiciel ImageJ, afin d'en améliorer le contraste. Les champs sont ensuite tous étudiés et les deux plus représentatifs de chaque condition sont présentés à chaque temps. L'analyse se fait par comparaison au contrôle.

### Résultats

### Viabilité cellulaire

**Tableau 4 : Viabilité cellulaire 24h après mise en contact**

| | queuine 0,1 µg/ml | queuine 1 µg/ml | queuine 10 µg/ml | queuine 100 µg/ml | Méthanol 0,0006% | Méthanol 0,006% | Méthanol 0,06% | Méthanol 0,6% |
|---|---|---|---|---|---|---|---|---|
| Moyennes des DO | 0,228 | 0,250 | 0,246 | 0,233 | 0,242 | 0,250 | 0,259 | 0,248 |
| | | | | | 10% SVF | 5% SVF | 0% SVF | Pas de cellules |
| | | | | | 0,273 | 0,303 | 0,187 | 0,095 |
| Moyenne - blanc | 0,133 | 0,155 | 0,151 | 0,138 | 0,146 | 0,154 | 0,164 | 0,153 |
| | | | | | 10% SVF | 5% SVF | 0% SVF | Pas de cellules |
| | | | | | 0,177 | 0,207 | 0,091 | 0,000 |
| Ecart-type | 0,007 | 0,006 | 0,011 | 0,004 | 0,014 | 0,023 | 0,019 | 0,010 |
| | | | | | 10% SVF | 5% SVF | 0% SVF | Pas de cellules |
| | | | | | 0,019 | 0,017 | 0,014 | 0,005 |

**Tableau 5 : Viabilité cellulaire 48h après mise en contact**

| | queuine 0,1 µg/ml | queuine 1 µg/ml | queuine 10 µg/ml | queuine 100 µg/ml | Méthanol 0,0006% | Méthanol 0,006% | Méthanol 0,06% | Méthanol 0,6% |
|---|---|---|---|---|---|---|---|---|
| Moyennes des DO | 0,304 | 0,331 | 0,328 | 0,273 | 0,329 | 0,315 | 0,324 | 0,345 |
| | | | | | 10% SVF | 5% SVF | 0% SVF | Pas de cellules |
| | | | | | 0,250 | 0,344 | 0,314 | 0,085 |
| Moyenne - blanc | 0,219 | 0,246 | 0,243 | 0,188 | 0,244 | 0,231 | 0,239 | 0,260 |
| | | | | | 10% SVF | 5% SVF | 0% SVF | Pas de cellules |
| | | | | | 0,165 | 0,259 | 0,229 | 0,000 |
| Ecart-type | 0,001 | 0,020 | 0,018 | 0,005 | 0,006 | 0,012 | 0,019 | 0,014 |
| | | | | | 10% SVF | 5% SVF | 0% SVF | Pas de cellules |
| | | | | | 0,011 | 0,011 | 0,035 | 0,004 |

**Tableau 6: Viabilité cellulaire 72h après mise en contact**

| | queuine 0,1 µg/ml | queuine 1 µg/ml | queuine 10 µg/ml | queuine 100 µg/ml | Méthanol 0,0006% | Méthanol 0,006% | Méthanol 0,06% | Méthanol 0,6% |
|---|---|---|---|---|---|---|---|---|
| Moyennes des DO | 0,341 | 0,362 | 0,395 | 0,272 | 0,386 | 0,400 | 0,362 | 0,376 |
| | | | | | 10% SVF | 5% SVF | 0% SVF | Pas de cellules |
| | | | | | 0,259 | 0,429 | 0,466 | 0,118 |
| Moyenne -blanc | 0,223 | 0,244 | 0,277 | 0,154 | 0,268 | 0,282 | 0,244 | 0,259 |
| | | | | | 10% SVF | 5% SVF | 0% SVF | Pas de cellules |
| | | | | | 0,141 | 0,311 | 0,348 | 0,000 |
| Ecart-type | 0,035 | 0,024 | 0,010 | 0,010 | 0,017 | 0,026 | 0,011 | 0,028 |
| | | | | | 10% SVF | 5% SVF | 0% SVF | Pas de cellules |
| | | | | | 0,008 | 0,020 | 0,015 | 0,001 |

### Interprétation des résultats

Les contrôles négatifs et positifs sont conformes et permettent de valider cette expérience. En effet, avec le test MTT, le contrôle 2,5% SVF est toujours supérieur au contrôle 10% SVF à 24h. Le contrôle 2,5% SVF est équivalent au contrôle 10% SVF à 48h et inférieur à celui-ci à 72h. Concernant le contrôle véhicule seul (gamme de méthanol de 0,6% à 0,0006%), celui-ci présente un léger effet cytotoxique, mais sans effet dose, et reste minime. Sa présence ne gêne donc pas la physiologie cellulaire, et ne sera pas un frein à la suite des expériences, même à sa plus haute concentration. Par ailleurs le méthanol est connu pour favoriser, au cours des premiers jours, la prolifération cellulaire.

En ce qui concerne la molécule queuine :
- À 48h, la concentration de 100 µg/ml présente un effet cytotoxique. Les DO obtenus sont similaires à celles du contrôle négatif (0% SVF).
- Les autres concentrations testées inférieures à 100 µg/ml ne présentent aucun effet cytotoxique.

### Prolifération cellulaire

**Tableau 7 : Viabilité cellulaire 24h après mise en contact**

| | queuine 0,3 µg/ml | queuine 1 µg/ml | queuine 3 µg/ml | queuine 10 µg/ml | queuine 30 µg/ml | Méthanol 0,0018% | Méthanol 0,006% | Méthanol 0,018% | Méthanol 0,06% | Méthanol 0,18% |
|---|---|---|---|---|---|---|---|---|---|---|
| Moyenne des DO | 1,312 | 1,328 | 1,522 | 1,625 | 1,634 | 1,609 | 1,554 | 1,489 | 1,446 | 1,475 |
| | | | | | | | 0% SVF | 2,5% SVF | 10% SVF | Pas de cellules |
| | | | | | | | 0,158 | 1,355 | 1,939 | 0,131 |
| Moyenne -blanc | 1,181 | 1,197 | 1,391 | 1,494 | 1,502 | 1,477 | 1,423 | 1,357 | 1,314 | 1,344 |
| | | | | | | | 0% SVF | 2,5% SVF | 10% SVF | Pas de cellules |
| | | | | | | | 0,027 | 1,223 | 1,808 | 0,000 |
| Ecart-type | 0,182 | 0,091 | 0,145 | 0,125 | 0,082 | 0,190 | 0,248 | 0,095 | 0,125 | 0,138 |
| | | | | | | | 0% SVF | 2,5% SVF | 10% SVF | Pas de cellules |
| | | | | | | | 0,003 | 0,042 | 0,126 | 0,027 |

À 48h et 72h le temps d'incubation en présence du substrat a été volontairement raccourci, afin que les DO ne soient pas au-delà du seuil de saturation du spectrophotomètre.

**Tableau 8 : Viabilité cellulaire 48h après mise en contact**

| | queuine 0,3 µg/ml | queuine 1 µg/ml | queuine 3 µg/ml | queuine 10 µg/ml | queuine 30 µg/ml | Méthanol 0,0018% | Méthanol 0,006% | Méthanol 0,018% | Méthanol 0,06% | Méthanol 0,18% |
|---|---|---|---|---|---|---|---|---|---|---|
| Moyenne des DO | 0,800 | 0,926 | 0,915 | 1,066 | 1,149 | 0,957 | 1,026 | 1,067 | 1,244 | 1,189 |
| | | | | | | | 0% SVF | 2,5% SVF | 10% SVF | Pas de cellules |
| | | | | | | | 0,107 | 0,937 | 1,517 | 0,123 |
| Moyenne -blanc | 0,677 | 0,803 | 0,793 | 0,943 | 1,026 | 0,834 | 0,903 | 0,944 | 1,122 | 1,066 |
| | | | | | | | 0% SVF | 2,5% SVF | 10% SVF | Pas de cellules |
| | | | | | | | -0,016 | 0,815 | 1,394 | 0,000 |
| Ecart-type | 0,068 | 0,126 | 0,129 | 0,050 | 0,143 | 0,083 | 0,108 | 0,113 | 0,068 | 0,016 |
| | | | | | | | 0% SVF | 2,5% SVF | 10% SVF | Pas de cellules |
| | | | | | | | 0,003 | 0,074 | 0,167 | 0,005 |

**Tableau 9 : Viabilité cellulaire 72h après mise en contact**

| | queuine 0,3 µg/ml | queuine 1 µg/ml | queuine 3 µg/ml | queuine 10 µg/ml | queuine 30 µg/ml | Méthanol 0,0018% | Méthanol 0,006% | Méthanol 0,018% | Méthanol 0,06% | Méthanol 0,18% |
|---|---|---|---|---|---|---|---|---|---|---|
| Moyenne des DO | 0,586 | 0,548 | 0,681 | 0,708 | 0,958 | 0,550 | 0,544 | 0,616 | 0,588 | 0,696 |
| | | | | | | | 0% SVF | 2,5% SVF | 10% SVF | Pas de cellules |
| | | | | | | | 0,133 | 0,499 | 1,159 | 0,170 |
| Moyenne -blanc | 0,416 | 0,378 | 0,511 | 0,538 | 0,787 | 0,380 | 0,374 | 0,446 | 0,418 | 0,525 |
| | | | | | | | 0% SVF | 2,5% SVF | 10% SVF | Pas de cellules |
| | | | | | | | -0,037 | 0,328 | 0,989 | 0,000 |
| Ecart-type | 0,033 | 0,108 | 0,009 | 0,041 | 0,061 | 0,061 | 0,075 | 0,070 | 0,082 | 0,019 |
| | | | | | | | 0% SVF | 2,5% SVF | 10% SVF | Pas de cellules |
| | | | | | | | 0,009 | 0,059 | 0,062 | 0,002 |

Le protocole utilisé est basé sur une incubation sans changement de milieu, ce qui induit un épuisement du milieu en facteur de croissance, particulièrement net pour le SVF choisi pour l'expérience (destiné à s'assurer qu'il ne contient pas de queuine, ce qui interférerait avec l'expérience). En conséquence la DO par unité de volume est plus faible que pour les temps plus courts.

### Interprétation des résultats

Les contrôles négatifs et positifs sont conformes et permettent de valider cette expérience.

En effet, avec le test BrdU, le contrôle 2,5% SVF est toujours inférieur au contrôle 10% SVF quel que soit le temps d'analyse (24, 48 et 72 heures).

Le témoin "méthanol" (connu pour stimuler la prolifération) présente :
- Un effet proche de celui de la queuine à 24 et 48 heures.
- Un effet inférieur à celui de la queuine à 72 heures et ce à partir de 3 µg/ml

Nous pouvons conclure que :
- La queuine n'a pas d'effet significatif dans des temps cours (24 et 48 heures) par rapport au véhicule.
- Un effet prolifératif net est mis en évidence à 72 heures pour les concentrations 3 et 10 µg/ml, particulièrement prononcé pour la concentration 30 µg/ml.

### Migration cellulaire

Les HDFa sont ensemencés en présence d'inserts de culture préalablement stérilisés, qui vont créer des zones acellulaires calibrées dans un tapis cellulaire confluent. Les cellules vont alors tendre à combler la brèche créée en migrant depuis les berges pour refermer la brèche.

Après 5h d'incubation à 37°C, les inserts sont retirés et les puits sont lavés deux fois en PBS 1X afin de retirer toute cellule n'ayant pas adhéré.

La queuine est ensuite ajoutée à 30, 10, 3 ou 1 µg/ml en DMEM 2,5% SVF, et une gamme équivalente de méthanol est également réalisée, deux puits par condition.

Des photos des puits à t=0h sont prises.

Les cellules sont ensuite cultivées 24h à 37°C, avant d'être fixées au paraformaldéhyde. Des photos à t=24h sont alors prises.

3 témoins sont aussi étudiés, 2 puits/témoin
- DMEM sans sérum,
- Milieu appauvri en sérum (DMEM supplémenté + 2,5% SVF)
- Milieu complet (DMEM supplémenté + 10% SVF).

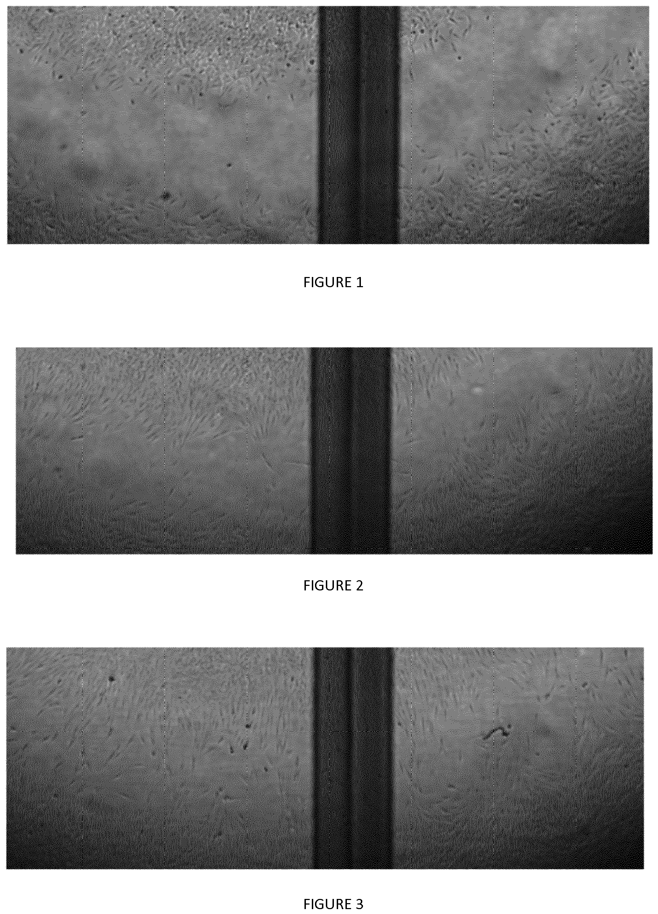

Les figures 1 et 2 montrent les effets du témoin DMEM 2,5% SVF (correspondant à 0 µg/ml de molécule à tester - témoin négatif) sur les fibroblastes, respectivement à 0 heure (figure 1) et 24 heures (figure 2).

La figure 3 montre les effets du témoin DMEM 10% SVF (correspondant au témoin positif) à 24 heures sur les fibroblastes.

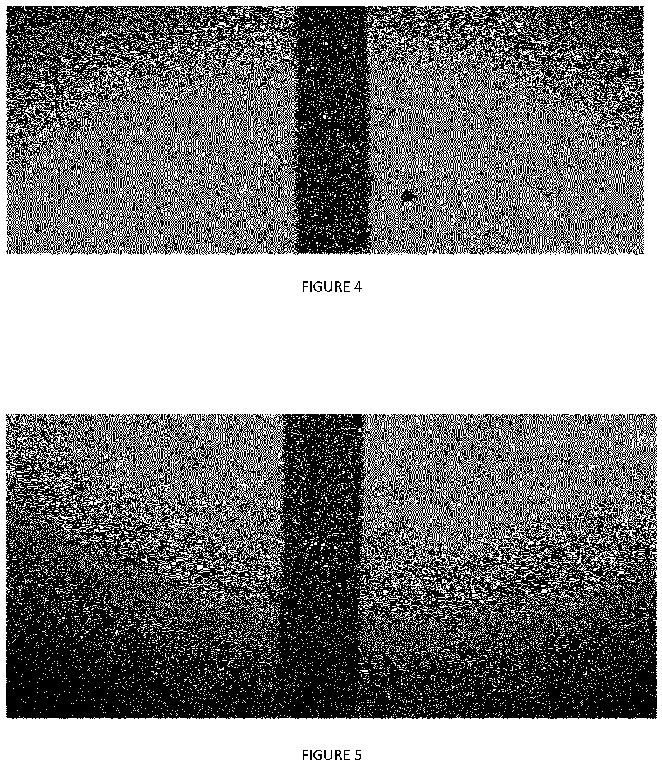

Les figures 4 et 5 montrent les effets de la queuine dans 2,5% SVF à 24 heures sur les fibroblastes, respectivement à une concentration de 10 µg/ml (figure 4) et 30 µg/ml (figure 5).

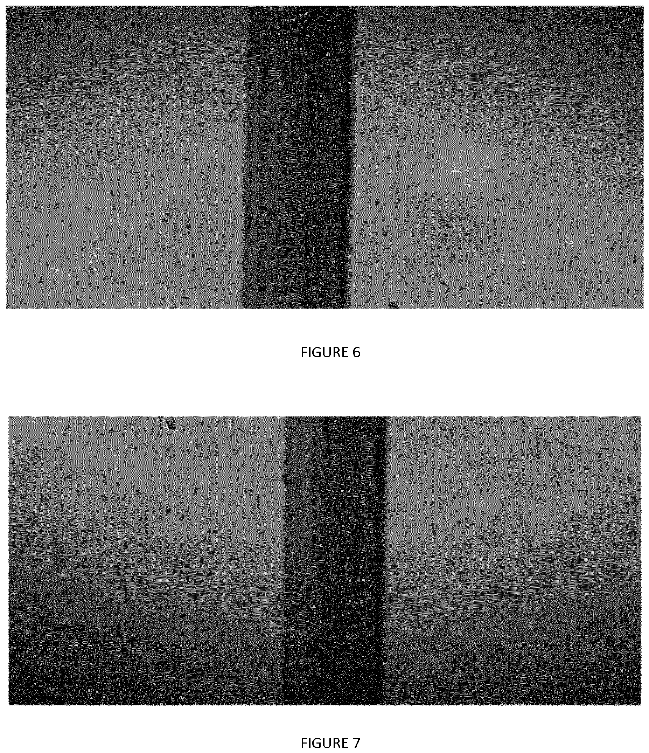

Les figures 6 et 7 montrent les effets du méthanol DMEM 2,5% SVF à 24 heures sur les fibroblastes, respectivement à une concentration de 10 µg/ml (figure 6) et 30 µg/ml (figure 7).

Le trait noir visible sur toutes les figures correspond à un marquage réalisé sur l'envers des boites pour identifier les zones à prendre en photos à 0h et 24h.

Les photos sont comparées entre elles, afin d'apprécier la différence de cellules ayant migré dans la brèche entre les différentes conditions testées.

On observe que, par rapport à la condition 0h, les cellules en 2,5% de SVF comme en 10% de SVF ont commencé à coloniser la brèche après 24h. Les cellules s'orientent en direction de la berge opposée, pour migrer dans cette direction, alors qu'au niveau du tapis cellulaire confluent visible, les extensions cytoplasmiques des fibroblastes n'ont pas d'orientation déterminé. De plus, il est observable sur ces images que plus de cellules ont migré dans la brèche avec 10% de SVF, il est notable que les cellules venant des deux berges opposées se sont rejointes au centre de la brèche.

L'observation des photos pour la queuine à 10 µg/ml et 30 µg/ml montre une migration supérieure à celle en 2,5% de SVF. Plus de cellules sont visibles dans la brèche, tout en conservant au niveau du front de migration l'orientation en navette vers la berge opposée visible en 2,5% de SVF. À 30 µg/ml enfin, les cellules des deux berges se sont rejointes au centre de la brèche, comme ce qui est visible en présence de 10% de SVF.

Les résultats montrent que :
- Les témoins 2,5% SVF et 10% de SVF présentent une migration proportionnelle au % de SVF mis en œuvre ;
- La queuine, a un effet sur la migration des fibroblastes et ce dès la concentration de 10µg/ml et que cet effet sur la migration des fibroblastes est confirmé à la concentration de 30µg/ml ;
- La migration en présence de la queuine à 10 et 30µg/ml est équivalente à celle obtenue avec le témoin positif SVF à 10%
- Le méthanol présente un léger effet sur la migration, mais est moins net que celui de la queuine.

### Conclusions générales

Les concentrations inférieures à 100 µg/ml de la queuine ne présentent aucun effet cytotoxique sur les HDFa. De plus, un effet prolifératif est mis en évidence avec la queuine aux concentrations suivantes 3, 10 et 30 µg/ml après 72 heures d'incubation. Un effet prolifératif important est observé avec la queuine, pour la concentration de 30 µg/ml à 72 heures. La queuine a un effet sur la migration des HDFa à la concentration de 10µg/ml. Cet effet est confirmé à la concentration de 30µg/ml. La migration en présence de la queuine à 10 et 30µg/ml est équivalente de celle obtenue avec le témoin positif SVF à 10%.

### Exemples de compositions

### Crème de base

6 volumes de Gel d'Aloe Vera (qui peut être remplacé par des infusions végétales, minérales ou de l'eau distillée), 2,5 volumes de mélange de beurre de karité et d'huile de jojoba (peut être remplacée par toute autre huile végétale ou de beurre) et 1,5 volumes de cire émulsifiante sont délicatement mélangés. Tous les ingrédients sont placés dans un bain marie pour permettre à la cire de fondre, puis vigoureusement mélangés et placés sur la glace pour refroidir. Puis, les principes actifs sont ajoutés. Enfin, le méthyle paraben est ajouté à une concentration finale de 0,19% à des fins de conservation.

De nombreux fabricants de crèmes cosmétiques de base neutres sont disponibles sur le marché (par exemple Neutra base ©), et toute base existante des grandes marques peut être améliorée par l'invention.

À cette base, la queuine est ajoutée à une concentration de 0,1 mg pour 100 ml.

Pour les préparations "bio", 100 mg d'extrait ARNt sont ajoutés à 100 ml de crème de base.

### Préparation des extraits d'ARN contenant la queuine

### Croissance bactérienne pour préparation biologique d'extraits contenant la queuine

De nombreux milieux de croissance sont adaptés. Par exemple, pour *Bacillus subtilis:*

### Milieu ED

Le milieu de croissance ED contient : K2HPO4, 8 mM; KH2PO4, 4,4 mM ; glucose, 27 mM ; Na3-citrate, 0,3 mM ; L-glutamine, 15 mM; citrate de fer ammoniacal, 33,5 microM ; MgSO4, 2 mM. Les oligoéléments sont rajoutés à cette base à partir d'une solution stock 100 fois concentrée pour une concentration finale de : MgCl2, 0,61 microM ; CaCl2, 49,5 microM ; FeCl3, 49,9 microM ; MnCl2, 5,05 microM ; ZnCl2, 12,4 microM ; CuCl2, 2,52 microM ; CoCl2, 2,5 microM ; Na2MoO4, 2,48 microM.

La souche sauvage de *Bacillus subtilis* est précultivée en milieu liquide ED à 37° C en aération constante. Une culture fraîche de la nuit est inoculée dans 15 ml de milieu ED à une densité optique de 0,1 à 600 nm (DO600). Les cellules sont cultivées à 37 ° C jusqu'à une DO600 de 1 et refroidies dans un volume égal de méthanol à 60% dans 70 mM de tampon HEPES pH 7,5 à -80 ° C. Toutes les étapes suivantes sont effectuées au froid et les solutions destinées à la préparation de l'ARN sont traitées avec du pyrocarbonate de diéthyle et stérilisées. Les cellules sont culottées à 4° C, lavées à l'eau et remises en suspension dans 0,5 ml de glucose à 10%, 11 mM de Tris-HCL Ph7,5, 10 mM d'EDTA. Les suspensions sont transférées dans des tubes contenant 0,1 g de billes de verre lavées à l'acide (Sigma-Aldrich, G4649).

Les tubes sont disposés dans l'adaptateur CoolPrep de l'instrument FastPrep ® -24 (MP Biomédical) contenant 50 g de carboglace. Les cellules sont cassées en trois cycles avec les paramètres suivants: 6 mètres par seconde pendant 45 s. Après chaque cycle, les suspensions sont conservées 1 min sur la glace. Après centrifugation 2 min à 10000 rpm, le surnageant est transféré dans un nouveau tube Eppendorf. L'acétate de sodium à pH 5,2 est ajouté à la concentration finale de 0,3 M et l'ARN total est isolé dans des conditions acides. Un volume de phénol acide:chloroforme et alcool isoamylique (125:24:1) à pH 4,5 (Amresco, AM9720) est ajouté. Chaque échantillon est mélangé au vortex 10s et incubé pendant 3 min dans un bain-marie à 65° C. Les phases sont séparées par centrifugation 5 min à 14000 rpm, puis la phase aqueuse est ré-extraite une fois avec la même procédure phénol acide chaud. La phase aqueuse est transférée dans un nouveau tube et complétée par un volume de phénol acide froid. Après centrifugation 5 min à 14000 rpm, l'ARN est précipité avec 2,5 volumes d'éthanol absolu 1 h à -80 ° C. L'ARN est centrifugé à 14.000 rpm pendant 15 min à 4° C et lavé avec de l'éthanol à 70%. Le culot d'ARN est dissous dans du Tris 10 mM, EDTA 1 mM pH 7,5. Il peut alors être utilisé comme première source d'extrait bactérien enrichi en queuine.

### Enrichissement en ARN de transfert

La préparation d'ARN total décrite précédemment est mélangée à un volume de chlorure de lithium 4,5 M pH 8 avec l'acétate de sodium pH 5,2 à la concentration finale de 0,01 mM. Cette solution d'ARN est incubée 2 heures à -80° C. Après centrifugation à 14000 rpm pendant 15 min à 4° C, l'ARNt se trouve dans le surnageant. Pour supprimer la contamination par le sel, l'ARNt est précipité 1h à -80 ° C par addition de 0,3 M d'acétate de sodium pH 5,2 et 2,5 volumes d'éthanol absolu. Ensuite, l'ARNt est sédimenté par centrifugation à 14000 rpm pendant 15 min à 4° C et lavé avec de l'éthanol à 70%. Le culot d'ARNt est dissous dans 10 mM de Tris, 1 mM EDTA pH 7,5. Cette préparation est une préparation d'ARN de transfert riche en queuine. Cette préparation peut ensuite être utilisée comme principe actif pour la préparation de composition cosmétique selon la présente invention.

## Revendications

1. Utilisation cosmétique de la queuine, ou d'un précurseur ou dérivé de celle-ci en tant que principe actif pour diminuer ou prévenir les signes de vieillissement de la peau et/ou des phanères, dans laquelle le précurseur ou dérivé de la queuine est sélectionné dans le groupe constitué de la queuosine, l'époxyqueuine, l'époxyqueuosine, la mannosylqueuine, la galactosylqueuine, la glutamylqueuine, la galactosylqueuosine, la mannosylqueuosine, la glutamylqueuosine et l'ARNt-queuosine.

2. Utilisation selon la revendication 1, dans laquelle la queuine, le précurseur ou dérivé de celle-ci, est destinée à diminuer ou prévenir les rides et ridules et/ou le flétrissement de la peau et/ou le manque d'élasticité et/ou de tonus de la peau et/ou l'amincissement de la peau et/ou le teint cireux et/ou parcheminé et/ou les irrégularités dans la texture de la peau et les irrégularités dans la pigmentation comme les taches de vieillesse.

3. Utilisation selon la revendication 1 ou 2, **caractérisée en ce que** la queuine, le précurseur ou dérivé de celle-ci est choisi dans le groupe consistant en la queuine, la queuosine, et l'epoxyqueuine.

4. Utilisation selon l'une quelconque des revendications 1 à 3, **caractérisée en ce que** la queuine, le précurseur ou dérivé de celle-ci est sous forme d'extrait bactérien ou d'extrait ou sève de plante, ledit extrait ou ladite sève étant riche ou enrichi(e) en queuine, précurseur ou dérivé de celle-ci.

5. Utilisation de queuine, ou d'un précurseur ou dérivé de celle-ci en tant que principe actif pour la préparation d'une composition cosmétique comprenant un milieu physiologiquement acceptable et destinée à diminuer ou prévenir les signes de vieillissement de la peau et/ou des phanères.

6. Utilisation selon la revendication 5, **caractérisée en ce que** la queuine, le précurseur ou dérivé de celle-ci est présent dans la composition dans une quantité de 0, 1 µg à 100 µg par ml ou g de composition cosmétique, de préférence de 1 à 5 µg par ml ou g de composition cosmétique.

7. Composition cosmétique comprenant, dans un milieu physiologiquement acceptable, de la queuine ou un précurseur ou dérivé de celle-ci en tant que principe actif **caractérisée en ce que** la queuine, le précurseur ou dérivé de celle-ci est présent dans la composition dans une quantité de 0,1 µg à 100 µg par ml ou g de composition cosmétique, de préférence de 1 à 5 µg par ml ou g de composition cosmétique et **en ce que** le précurseur ou dérivé de la queuine est sélectionné dans le groupe constitué de la queuosine, l'époxyqueuine, l'époxyqueuosine, la mannosylqueuine, la galactosylqueuine, la glutamylqueuine, la galactosylqueuosine, la mannosylqueuosine, la glutamylqueuosine et l'ARNt-queuosine.

8. Composition cosmétique selon la revendication 7, **caractérisée en ce que** la queuine, le précurseur ou dérivé de celle-ci est choisi dans le groupe consistant en la queuine, la queuosine, et l'époxyqueuine.

9. Composition cosmétique selon la revendication 7, **caractérisée en ce qu'**elle comprend un extrait bactérien ou un extrait ou une sève de plante, ledit extrait ou ladite sève étant riche ou enrichi(e) en queuine, précurseur ou dérivé de celle-ci.

10. Composition cosmétique selon l'une quelconque des revendications 7 à 9, **caractérisée en ce que** la queuine, le précurseur ou dérivé de celle-ci est présent dans la composition avec des co-facteurs ou vitamines, comme par exemple la vitamine C ou le tocophérol.

11. Composition cosmétique selon l'une quelconque des revendications 7 à 10, **caractérisée en ce qu'**elle se présente sous forme de sérum, de lotion, de crème, de lait, de gel aqueux ou huileux, d'hydrogel, de masque, de bâton, de patch, d'huile, d'onguent, de cire, d'une mousse, de tonique, d'eau de soin, de baume, de fond de teint, de spray, d'ombre à paupière, de crème amincissante, de rouge à lèvre, d'une pâte, d'une pommade ou d'un shampoing ou après-shampoing.

12. Composition cosmétique selon l'une quelconque des revendication 7 à 11, **caractérisée en ce qu'**elle est formulée pour une application topique.

13. Procédé de traitement cosmétique pour prévenir et/ou traiter les signes de vieillissement de la peau et/ou des phanères, comprenant l'application sur la peau et/ou les phanères d'une composition cosmétique selon l'une des revendications 7 à 12.

## Patentansprüche

1. Kosmetischer Gebrauch von Queuin oder eines Zwischenstoffes oder Derivates davon als Wirkstoff zur Verringerung und Vorbeugung von Alterungserscheinungen der Haut und/oder der Hautanhangsgebilde, in dem der Zwischenstoff oder das Derivat von Queuin aus der Gruppe, bestehend aus Queuosin, Epoxyqueuin, Epoxyqueuosin, Mannosylqueuin, Galaktosylqueuin, Glutamylqueuin, Galaktosylqueuosin, Mannosylqueuosin, Glutamylqueuosin und tRNA-Queuosin, gewählt wurde.

2. Gebrauch nach Patentanspruch 1, in dem das Queuin, der Zwischenstoff oder das Derivat davon dazu bestimmt ist, Runzeln und Fältchen und/oder das Welken der Haut und/oder fehlende Elastizität und/oder Spannung der Haut und/oder Verdünnung der Haut und/oder wachs- oder pergamentartigen Teint und/oder Unregelmäßigkeiten in der Struktur der Haut und die Unregelmäßigkeiten der Pigmentation, wie etwas Altersflecken zu verringern oder ihnen vorzubeugen.

3. Gebrauch nach Patentanspruch 1 oder 2, **dadurch gekennzeichnet, dass** das Queuin, der Zwischenstoff oder das Derivat davon aus der Gruppe, bestehend aus Queuin, Queuosin und Epoxyqueuin gewählt wurde.

4. Gebrauch nach irgendeinem der Patentansprüche 1 bis 3, **dadurch gekennzeichnet, dass** das Queuin, der Zwischenstoff oder das Derivat davon als Bakterienextrakt oder Pflanzenextrakt oder -saft vorliegt, wobei der genannte Extrakt oder der genannte Saft an Queuin, Zwischenstoff oder Derivat davon reich oder angereichert ist.

5. Gebrauch von Queuin oder eines Zwischenstoffes oder Derivates davon als Wirkstoff zur Bereitung einer kosmetischen Zubereitung, die ein physiologisch verträglich Medium umfasst und dazu bestimmt ist, Alterungserscheinungen der Haut und/oder der Hautanhangsgebilde zu verringern oder ihnen vorzubeugen.

6. Gebrauch nach Patentanspruch 5, **dadurch gekennzeichnet, dass** das Queuin, der Zwischenstoff oder das Derivat davon in der Zubereitung in einer Menge von 0,1 µg bis 100 µg pro ml oder g der kosmetischen Zubereitung vorliegt, vorzugsweise von 1 bis 5 µg pro ml oder g der kosmetischen Zubereitung.

7. Kosmetische Zubereitung, in einem physiologisch verträglichen Medium Queuin oder einen Zwischenstoff oder ein Derivat davon als Wirkstoff umfassend, **dadurch gekennzeichnet, dass** das Queuin, der Zwischenstoff oder das Derivat davon in der Zubereitung in einer Menge von 0,1 µg bis 100 µg pro ml oder g der kosmetischen Zubereitung, vorzugsweise von 1 bis 5 µg pro ml oder g der kosmetischen Zubereitung, vorliegt und dadurch, dass der Zwischenstoff oder das Derivat von Queuin aus der Gruppe, bestehend aus Queuosin, Epoxyqueuin, Epoxyqueuosin, Mannosylqueuin, Galaktosylqueuin, Glutamylqueuin, Galaktosylqueuosin, Mannosylqueuosin, Glutamylqueuosin und tRNA-Queuosin, gewählt wurde.

8. Kosmetische Zubereitung nach Patentanspruch 7, **dadurch gekennzeichnet, dass** das Queuin, der Zwischenstoff oder das Derivat davon aus der Gruppe, bestehend aus Queuin, Queuosin und Epoxyqueuin gewählt wurde.

9. Kosmetische Zubereitung nach Patentanspruch 7, **dadurch gekennzeichnet, dass** sie einen Bakterienextrakt oder Pflanzenextrakt oder-saft enthält, wobei der genannte Extrakt oder der genannte Saft an Queuin, Zwischenstoff oder Derivat davon reich oder angereichert ist.

10. Kosmetische Zubereitung nach irgendeinem der Patentansprüche 7 bis 9, **dadurch gekennzeichnet, dass** das Queuin, der Zwischenstoff oder das Derivat davon in der Zubereitung mit Kofaktoren oder Vitaminen, wie beispielsweise Vitamin C oder Tocopherol, vorliegt.

11. Kosmetische Zubereitung nach irgendeinem der Patentansprüche 7 bis 10, **dadurch gekennzeichnet, dass** sie die Form von Serum, Lotion, Creme, Milch, wässrigem oder öligem Gel, Hydrogel, Maske, Stift, Pflaster, Öl, Salbe, Wachs, Schaum, Tonikum, Pflegewasser, Balsam, Make-Up-Grundierung, Spray, Lidschatten, Abmagerungscreme, Lippenstift, Paste, Pomade oder Shampoo oder Spülung aufweist.

12. Kosmetische Zubereitung nach irgendeinem der Patentansprüche 7 bis 11, **dadurch gekennzeichnet, dass** sie für lokale Anwendung formuliert ist.

13. Kosmetisches Behandlungsverfahren zur Verringerung und/oder Vorbeugung von Alterungserscheinungen der Haut und/oder der Hautanhangsgebilde, das Auftragen einer kosmetischen Zubereitung nach einem der Patentansprüche 7 bis 12 auf die Haut und/oder der Hautanhangsgebilde umfassend.

## Claims

1. Cosmetic use of queuine, or a precursor or derivative thereof as an active principle for reducing or preventing signs of skin aging and/or skin appendages, wherein the precursor or derivative of queuine is selected from the group consisting of queuosine, epoxyqueuine, epoxyqueuosine, mannosylqueuine, galactosylqueuine, glutamylqueuine, galactosylqueuosine, mannosylqueuosine, glutamylqueuosine and queuosine-tRNA.

2. Use according to claim 1, wherein queuine, the precursor or derivative thereof, is intended to reduce or prevent wrinkles and fine lines et/or skin wilting and/or lack of elasticity et/or skin tone and/or skin thinning and/or waxy complexion and/or parchment complexion and/or irregularities in the skin texture and irregularities in pigmentation such as age spots.

3. Use according to claim 1 or 2, **characterised in that** queuine, the precursor or derivative thereof is selected from the group consisting of queuine, queuosine, and epoxyqueuine.

4. Use according to any one of claims 1 to 3, **characterized in that** queuine, the precursor or derivative thereof is in the form of bacterial extract or plant extract or sap, said extract or said sap being rich or enriched in queuine, the precursor or derivative thereof.

5. Use of queuine, or a precursor of derivative thereof as an active principle for the preparation of a cosmetic composition comprising a physiologically acceptable medium and intended to reduce or prevent signs of skin aging and/or skin appendages.

6. Use according to claim 5, **characterized in that** queuine, the precursor or derivative thereof is present in the composition in an amount of from 0,1 µg to 100 µg per ml or g of cosmetic composition, preferably from 1 to 5 µg per ml or g of cosmetic composition.

7. Cosmetic composition comprising, in a physiologically acceptable medium, queuine or a precursor or derivative thereof as an active principle **characterized in that** queuine, the precursor or derivative thereof is present in the composition in an amount of from 0.1 µg to 100 µg per ml or g of cosmetic composition, preferably from 1 to 5 µg per ml or g of cosmetic composition and **in that** the precursor or derivative of queuine is selected from the group consisting of queuosine, epoxyqueuine, epoxyqueuosine, mannosylqueuine, galactosylqueuine, glutamylqueuine, galactosylqueuosine, mannosylqueuosine, glutamylqueuosine and queuosine-tRNA.

8. Cosmetic composition according to claim 7, **characterized in that** queuine, the precursor or derivative thereof is selected from the group consisting of queuine, queuosine and epoxyqueuine.

9. Cosmetic composition according to claim 7, **characterized in that** it comprises a bacterial extract or a plant extract or sap, said extract or said sap being rich or enriched in queuine, a precursor or derivative thereof.

10. Cosmetic composition according to any one of claims 7 to 9, **characterized in that** queuine, the precursor or derivative thereof is present in the composition with cofactors or vitamins, such as for example vitamin C or tocopherol.

11. Cosmetic composition according to any one of claims 7 to 10, **characterized in that** it is in the form of serum, lotion, cream, milk, aqueous or oily gel, hydrogel, mask, stick, patch, oil, ointment, wax, mousse, toner, skin care water, balm, foundation, spray, eye shadow, slimming cream, lipstick, paste, ointment or shampoo or conditioner.

12. Cosmetic composition according to any one of claims 7 to 11, **characterized in that** it is formulated for topical application.

13. Cosmetic treatment process for preventing and/or treating signs of skin aging and/or skin appendages, comprising the application to the skin and/or skin appendages of a cosmetic composition according to any one of claims 7 to 12.
